# FASCICULE DE BREVET EUROPEEN

(11) **EP 1 629 079 B1**
(45) Date de publication et mention de la délivrance du brevet: **22.01.2014**
(21) Numéro de dépôt: 04767250.6
(22) Date de dépôt: 03.06.2004
(51) Int. Cl.: B01D 45/12, G01N 1/22, G01N 1/24, G01N 33/00

(54) **DISPOSITIF DE COLLECTE ET DE SEPARATION DE PARTICULES ET DE MICRO-ORGANISMES PRESENTS DANS L'AIR AMBIANT**
VORRICHTUNG ZUM SAMMELN UND TRENNEN VON IN DER UMGEBUNGSLUFT VORHANDENEN PARTIKELN UND MIKROORGANISMEN
DEVICE FOR COLLECTING AND SEPARATING PARTICLES AND MICROORGANISMS PRESENT IN THE AMBIENT AIR

(30) Priorité: 04.06.2003 FR 0306749
(43) Date de publication de la demande: 01.03.2006
(73) Titulaire: Bertin Technologies S.A., 78180 Montigny-le-Bretonneux (FR)
(72) Inventeur: VALLAYER, Bruno, F-33000 Bordeaux (FR); TROUCHET, Daniel, F-75015 Paris (FR); VERDIER, Amandine, F-78000 Versailles (FR); SOREL, Emmanuelle, F-28260 ROUVRES (FR)
(74) Mandataire: Ramey, Daniel
(86) Numéro de dépôt international: PCT/FR2004/001382
(87) Numéro de publication internationale: WO 2004/108880

(56) Documents cités:
- CA-A- 2 151 893
- US-A- 5 500 369
- US-A- 5 902 385

## Description

L'invention concerne un dispositif de collecte et de séparation de particules et de micro-organismes présents dans l'air ambiant, à des fins d'identification et de dénombrement de ces particules et micro-organismes.

L'évaluation qualitative et quantitative des particules et micro-organismes dans l'air est importante dans de nombreux domaines tels que l'industrie pharmaceutique, l'industrie agroalimentaire, le milieu médical, les services d'hygiène, les services vétérinaires, etc. Les dimensions des particules et micro-organismes à collecter peuvent varier de 0,5 à 15 µm environ.

Les particules biologiques présentes dans l'air comprennent notamment des bactéries, des moisissures, des virus, des pollens, ... et sont en général collectées par impact sur une gélose nutritive ou sélective, ou par impact sur une surface liquide ou encore par centrifugation en milieu liquide.

La collecte de ces particules ou micro-organismes par impact sur gélose est suivie d'une incubation et les colonies obtenues par culture sont dénombrées et identifiées. Un inconvénient de ce procédé est que seuls les micro-organismes viables et cultivables sur le milieu utilisé peuvent être dénombrés, mais non les micro-organismes qui sont déjà stressés lors de la collecte ou qui le deviennent à la suite de l'aspiration ou de l'impact sur la gélose (US 5 902 385).

Ce procédé ne peut donc être exécuté qu'avec de très faibles débits d'aspiration d'air et pendant une courte durée pour ne pas endommager les micro-organismes collectés et aussi pour ne pas dessécher la gélose, ces débits d'air étant typiquement inférieurs à 100 litres par minute. La charge microbienne mesurée avec ce procédé est en général sous-évaluée.

Dans le cas de la collecte par impact sur une surface liquide, les particules présentes dans l'air aspiré sont projetées sur des parois humides d'une chambre et récupérées en partie inférieure de la chambre sous forme d'échantillon liquide. Un inconvénient de ce procédé est le bullage du liquide provoqué par le débit d'air et la re-dispersion des particules par rebond sur les parois de la chambre.

Dans le cas de la collecte par centrifugation en milieu liquide, les particules sont aspirées dans une chambre cylindrique où elles sont mises en rotation puis tombent dans un tube contenant un liquide.

Les plus grosses particules sont plaquées sur les parois de la chambre et récupérées en partie basse tandis que les particules les plus fines sont emportées vers la sortie par le débit d'air et ne sont pas collectées. Dans les systèmes existants, les débits d'air utilisés sont relativement élevés pour assurer une collecte suffisante de particules et de micro-organismes et les dimensions et le poids des matériels utilisés (chambre cyclonique, moyens d'aspiration d'air) sont relativement importants de sorte que ces matériels sont fixes et installés à demeure, ce qui limite leur utilisation.

En outre, les particules collectées sont récupérées en général dans un flacon à l'extrémité inférieure de la chambre cylindrique, puis ce flacon est fermé au moyen d'un bouchon. Cette façon de faire ne garantit pas, d'une part, que toutes les particules séparées sont bien collectées dans le flacon et n'évite pas, d'autre part, les risques de contamination quand le flacon est séparé de la chambre cyclonique.

L'invention a pour objet un dispositif de collecte et de séparation de particules et de micro-organismes présents dans l'air ambiant qui combine les avantages des techniques connues sans en présenter les inconvénients.

Elle propose, à cet effet, un dispositif de collecte de particules et de micro-organismes présents dans l'air ambiant selon la revendication 1.

Ainsi, selon l'invention, c'est le même élément qui sert à la séparation et à la collecte des particules et des micro-organismes présents dans l'air ambiant et à leur transport aux fins d'analyse. Cela réduit les manipulations et diminue les risques de contamination des particules et micro-organismes collectés. Cela garantit également que les particules et micro-organismes analysés représentent 100% des particules et micro-organismes collectés.

Selon une autre caractéristique de l'invention, l'enceinte comprend des moyens d'obturation automatique des orifices de son extrémité supérieure, ces moyens étant ouverts quand l'enceinte est montée sur le dispositif et étant fermés quand elle est retirée du dispositif.

Par exemple, ces moyens d'obturation automatique comprennent un diaphragme annulaire et des moyens de rappel élastique du diaphragme en position de fermeture de l'extrémité supérieure ouverte de l'enceinte. D'autres moyens sont prévus si nécessaire pour fermer un ou des orifices d'entrée d'air dans l'enceinte.

Cette obturation automatique de l'enceinte à son démontage de sur le dispositif évite les risques de contamination des particules et micro-organismes collectés.

L'enceinte peut être montée sur le dispositif par encliquetage élastique et par rotation quart de tour, le mouvement de translation de l'enceinte lors de son encliquetage ou sa rotation quart de tour assurant sans difficulté l'ouverture ou la fermeture des moyens d'obturation automatique précités.

Le dispositif selon l'invention a pour avantage essentiel de pouvoir être utilisé en tout endroit, grâce à son autonomie, son faible volume et son faible poids qui permettent non seulement de le transporter facilement mais aussi de le manipuler d'une main, de le placer et de l'orienter à volonté.

On peut ainsi, grâce à ce dispositif, effectuer des prélèvements successifs en plusieurs endroits et en orientant le dispositif de façon différente d'un endroit à l'autre, par exemple dans le même local. L'opérateur peut en particulier être muni d'un équipement comportant plusieurs enceintes de rechange, ce qui lui permet de faire plusieurs prélèvements de suite dans un laps de temps court.

De préférence, l'enceinte amovible est à usage unique, ou bien elle est réalisée en matériau autoclavable.

Dans un premier mode de réalisation de l'invention, la collecte et la récupération des particules et micro-organismes dans l'enceinte ont lieu à sec et un liquide approprié est ensuite ajouté en quantité prédéterminée dans cette partie inférieure pour l'obtention d'un échantillon liquide que l'on peut ensuite traiter de la façon voulue.

Dans un autre mode de réalisation de l'invention, le dispositif comprend des moyens d'injection de liquide en partie supérieure de l'enceinte montée sur le dispositif, ces moyens d'injection débouchant par exemple dans des moyens d'entrée d'air dans l'enceinte.

On obtient ainsi directement, dans la partie inférieure de l'enceinte, un échantillon liquide contenant les particules et micro-organismes collectés.

Dans une forme de réalisation particulièrement avantageuse, le dispositif selon l'invention comprend une poignée qui est reliée à la partie fixe de support de l'enceinte et/ou aux moyens d'aspiration d'air et qui contient un réservoir de liquide, des moyens de mise en pression du liquide dans le réservoir et des moyens de liaison du réservoir à des moyens d'injection dans l'enceinte précitée.

Avantageusement, ces moyens de liaison comprennent des moyens de commande du débit de liquide vers les moyens d'injection.

De plus, cette poignée comprend des moyens de commande des moyens d'aspiration d'air.

Avec le dispositif selon l'invention, un prélèvement peut durer quelques minutes et permet de collecter les particules et micro-organismes présents dans un volume d'air ambiant compris entre 1 et 2 m³.

Le dispositif selon l'invention permet de séparer et de collecter des particules et micro-organismes ayant des dimensions comprises typiquement entre 0,5 et 15 µm environ. Pour la collecte de particules plus fines dont les dimensions sont inférieures à 1 µm environ, un filtre approprié peut être placé dans la sortie d'air axiale de l'enceinte précitée.

L'invention sera mieux comprise et d'autres caractéristiques, détails et avantages de celle-ci apparaîtront plus clairement à la lecture de la description qui suit, faite à titre d'exemple en référence aux dessins annexés dans lesquels :
- la figure 1 est une vue schématique en perspective d'un premier mode de réalisation d'un dispositif selon l'invention ;
- la figure 2 est un schéma fonctionnel de ce dispositif ;
- les figures 3a et 3b sont des vues schématiques illustrant l'utilisation de ce dispositif ;
- la figure 4 est une vue schématique fonctionnelle d'une variante de réalisation de l'invention.

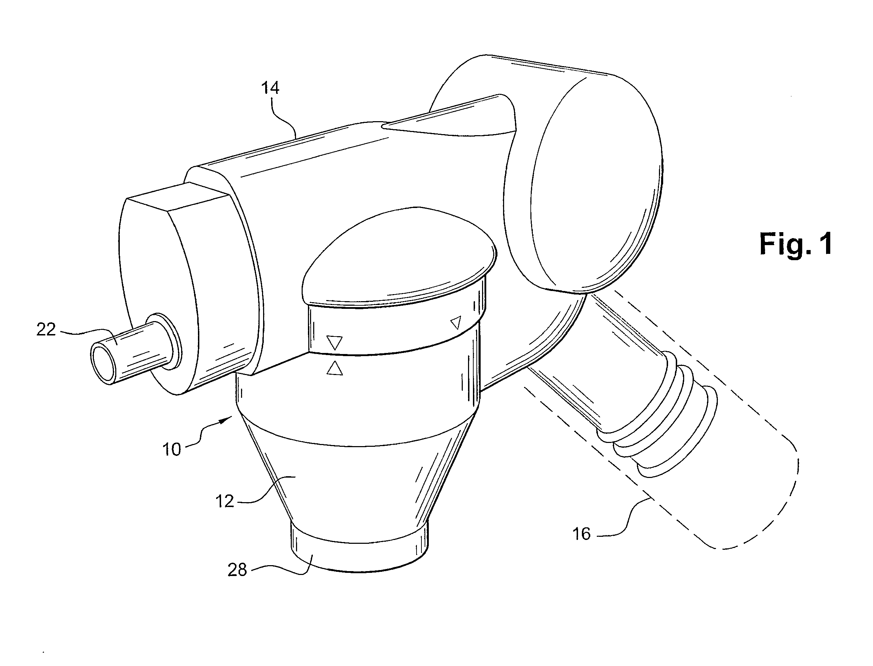

On se réfère d'abord aux figures 1 à 3 pour décrire un premier mode de réalisation du dispositif selon l'invention.

Ce dispositif est autonome, portable et manipulable d'une seule main et comprend essentiellement une enceinte cylindrique 10 montée sur une partie fixe du dispositif et comportant un corps cylindrique 13 et une partie inférieure 12 de forme tronconique, l'enceinte 10 délimitant une chambre de centrifugation et étant reliée à des moyens 14 d'aspiration d'air. Le dispositif est équipé d'une poignée 16 munie de moyens de commande du fonctionnement des moyens d'aspiration d'air, ces moyens de commande étant par exemple du type à bouton-poussoir ou à curseur.

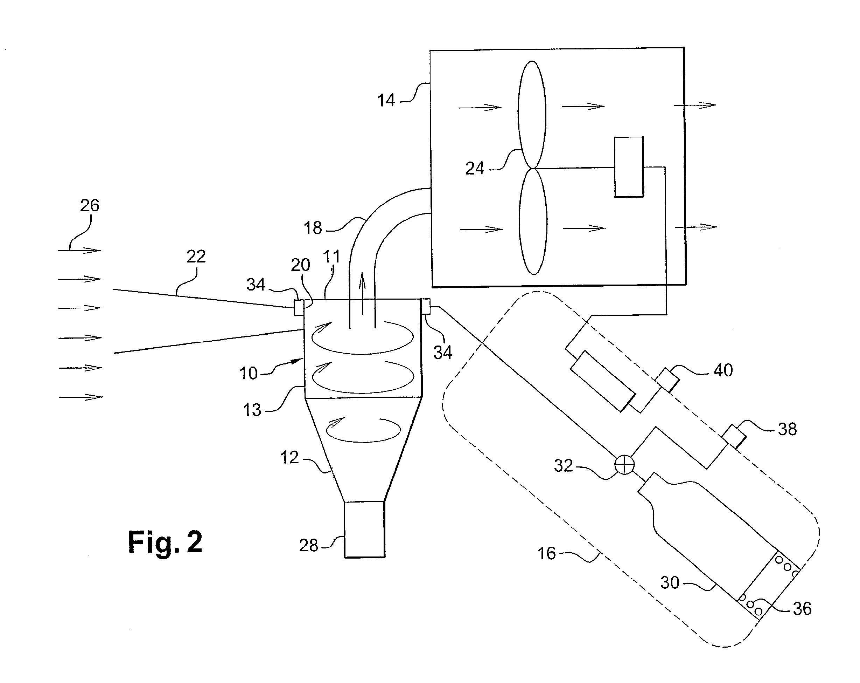

La partie fixe du dispositif sur laquelle est montée l'enceinte comprend une paroi supérieure 11 avec une sortie d'air axiale 18 et un rebord cylindrique 20 orienté vers le bas et qui comprend un orifice relié à un conduit d'entrée d'air 22 débouchant tangentiellement dans l'enceinte 10. La paroi 11 est solidaire des moyens d'aspiration 14 et la sortie d'air axiale 18 est reliée à un volume d'aspiration ménagé dans le boîtier des moyens 14 et contenant un rotor 24 entraîné par un moteur électrique et dont la rotation provoque par aspiration dans le conduit 18, une entrée d'air dans l'enceinte 10 par le conduit 22 et une mise en rotation de l'air à l'intérieur de l'enceinte comme indiqué par les flèches en figure 2.

L'air ambiant 26 est ainsi aspiré dans l'enceinte 10 et les particules et micro-organismes qu'il contient sont séparés par centrifugation et déposés sur la paroi interne de l'enceinte 10 puis récupérés dans la partie inférieure 12 tronconique dont la section se réduit vers le bas et peut comporter un embout cylindrique 28 de fermeture.

Avantageusement, le corps cylindrique 13 et la partie tronconique 12 de l'enceinte forment un ensemble monobloc monté de façon amovible sur le rebord cylindrique 20, par encliquetage élastique ou par rotation quart de tour.

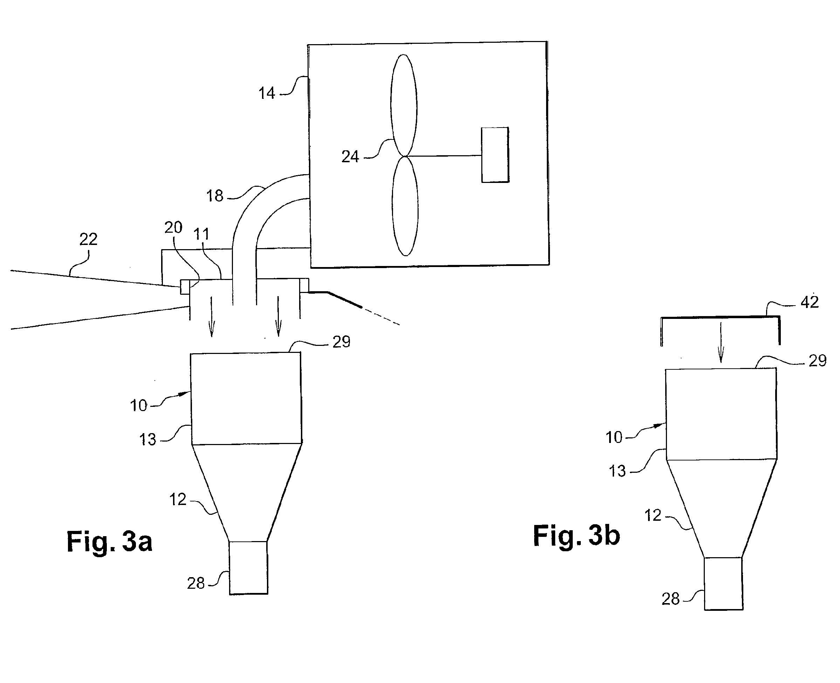

L'orifice d'entrée d'air et l'extrémité supérieure ouverte de l'enceinte 10 peuvent être obturées par un capuchon 42 quand l'enceinte est démontée du dispositif, comme représenté en figure 3a et 3b, l'enceinte fermée formant alors un récipient qui permet de conserver les particules et micro-organismes collectés et de les transporter vers un laboratoire d'analyses.

En variante, l'extrémité supérieure de l'enceinte peut être équipée d'un moyen d'obturation automatique 29, tel par exemple qu'un diaphragme annulaire du type de ceux utilisés dans les appareils photographiques, qui est associé à un ressort de rappel et qui est actionné en ouverture quand l'enceinte 10 est montée sur le dispositif et en fermeture quand cette enceinte est retirée du dispositif.

Cette fermeture automatique de l'extrémité supérieure de l'enceinte 10 à son retrait du dispositif évite tout risque de contamination des micro-organismes et particules collectés dans l'enceinte 10 par des agents extérieurs, ou inversement, tout risque de contamination du milieu extérieur par les micro-organismes et particules collectés dans l'enceinte 10. Des moyens appropriés sont prévus pour obturer l'orifice d'entrée d'air dans l'enceinte.

Pour plus de sécurité, l'extrémité supérieure de l'enceinte 10 fermée par le moyen d'obturation automatique 29 peut être coiffée d'un capuchon 42 comme représenté en figure 3b pour son stockage et son transport vers des moyens d'analyse.

Dans le mode de réalisation représenté en figure 2, la poignée 16 du dispositif contient également un réservoir 30 de liquide approprié (de l'eau et un tensioactif, ou bien un autre liquide en fonction des analyses à effectuer), dont la sortie comprend des moyens 32 de réglage de débit reliés à des moyens 34 d'injection dans la partie supérieure de l'enceinte 10.

Par exemple, ces moyens d'injection 34 débouchent dans le conduit 22 d'amenée d'air dans l'enceinte.

En variante, la partie supérieure fixe de l'enceinte comprend plusieurs orifices d'injection de liquide, orientés tangentiellement et répartis de façon régulière sur une circonférence du rebord cylindrique 20 et qui débouchent dans l'enceinte à travers des ouvertures de la partie supérieure de l'enceinte 10.

Le réservoir 30 est avantageusement associé à des moyens 36 de mise en pression du liquide à injecter, ces moyens 36 étant de tout type approprié, par exemple comprenant un ressort agissant sur un fond mobile du réservoir 30 comme représenté, un gaz sous pression, etc... Le réservoir 30 peut lui-même être "monodose", c'est-à-dire qu'il contient une quantité de liquide relativement faible suffisante pour un prélèvement, ou bien "multidoses" et comprend alors une quantité de liquide plus importante permettant plusieurs prélèvements successifs.

Les moyens 32 de réglage de débit d'injection sont commandés par un curseur ou un bouton-poussoir 38 porté par la poignée 16, au voisinage d'un autre bouton-poussoir ou curseur 40 de commande du fonctionnement des moyens d'aspiration d'air 14.

La poignée contient également des moyens d'alimentation électrique du moteur électrique d'entraînement du rotor 24, ces moyens étant par exemple des accumulateurs rechargeables ou analogues. En variante, ce moteur électrique peut être alimenté par connexion à un réseau électrique.

Dans un mode de réalisation particulier de l'invention, l'enceinte 10 a un diamètre de l'ordre de 50 millimètres, le volume de cette enceinte y compris la partie inférieure 12 est de l'ordre de 100 à 200 cm³, le débit d'air aspiré dans l'enceinte 10 par les moyens 14 est compris entre 200 et 400 litres par minute, le volume de liquide injecté dans l'enceinte 10 par les moyens 34 à chaque prélèvement est compris entre 5 et 10 ml environ, le réservoir 30 contient par exemple 40 ml de liquide (ce qui permet de 4 à 8 prélèvements), la puissance électrique installée est de 100 à 200 W, et le poids total du dispositif est inférieur à 2 kg.

Ce dispositif est utilisé de la façon suivante :

Il peut être tenu à la main et orienté dans une direction déterminée ou bien posé sur un support. Pour un prélèvement, l'enceinte 10 est fixée sur le rebord cylindrique 20 et l'on commande le fonctionnement des moyens 14 d'aspiration d'air au moyen du bouton-poussoir 40. La durée du prélèvement est par exemple de 5 minutes, ce qui permet d'aspirer de 1 à 2 m³ d'air environ, l'air aspiré circulant dans l'enceinte 10 en tournant autour de son axe puis en remontant pour sortir par le conduit 18 et passer dans le boîtier des moyens 14 d'aspiration d'air pour être éjecté à l'extérieur. L'injection de liquide peut être continue pendant toute la durée du prélèvement, ou bien elle peut avoir lieu pendant une partie du prélèvement.

Les particules et micro-organismes véhiculés par l'air aspiré sont centrifugés sur la paroi interne de l'enceinte 10 et sont ainsi séparés de l'air aspiré qui ressort par le conduit 18. Le liquide injecté dans l'enceinte 10 circule comme l'air aspiré et effectue un lavage complet de la surface interne de cette enceinte. Les particules et micro-organismes collectés sont finalement récupérés dans la partie inférieure 12 et dans l'embout cylindrique 28.

A la fin d'un prélèvement, comme représenté aux figures 3a et 3b, on peut retirer par déclipsage ou par rotation quart de tour l'enceinte 10 de sur le rebord cylindrique supérieur 20 puis on coiffe le corps 13 de l'enceinte 10 du capuchon 42 qui ferme tous les orifices de la partie supérieure de l'enceinte 10. On obtient ainsi un petit récipient fermé de façon étanche que l'on peut conserver à des fins d'analyse.

Pour poursuivre les prélèvements, il suffit de mettre en place une autre enceinte 10 sur le rebord cylindrique supérieur 20.

Les faibles dimensions du dispositif selon l'invention permettent de le transporter avec une série de 4 ou 5 enceintes amovibles dans une mallette, qui peut également contenir un ou deux réservoirs 30 de rechange ainsi qu'éventuellement un deuxième accumulateur rechargeable pour l'alimentation du moteur d'entraînement des moyens d'aspiration d'air 14.

En variante, les moyens 14 d'aspiration d'air peuvent comprendre un petit réservoir d'air comprimé, qui va permettre une aspiration d'air dans l'enceinte cylindrique 10 par entraînement. Le dispositif peut alors être utilisé sans risque de déflagration causée par une étincelle électrique.

Dans une autre variante, on peut également réaliser l'aspiration d'air dans l'enceinte cylindrique 10 en connectant le dispositif selon l'invention à une prise d'aspiration, du type de celles dont certains bâtiments et hôpitaux sont équipés.

Selon un autre aspect de l'invention, le réservoir de liquide 30 et ses moyens de pressurisation 36 peuvent être constitués par une seringue d'injection à commande manuelle ou semi-automatique.

Le dispositif selon l'invention peut également être utilisé pour effectuer des prélèvements et des séparations de particules et micro-organismes à sec, les particules et micro-organismes prélevés et collectés étant ensuite mis en solution dans un liquide approprié aux fins d'analyses.

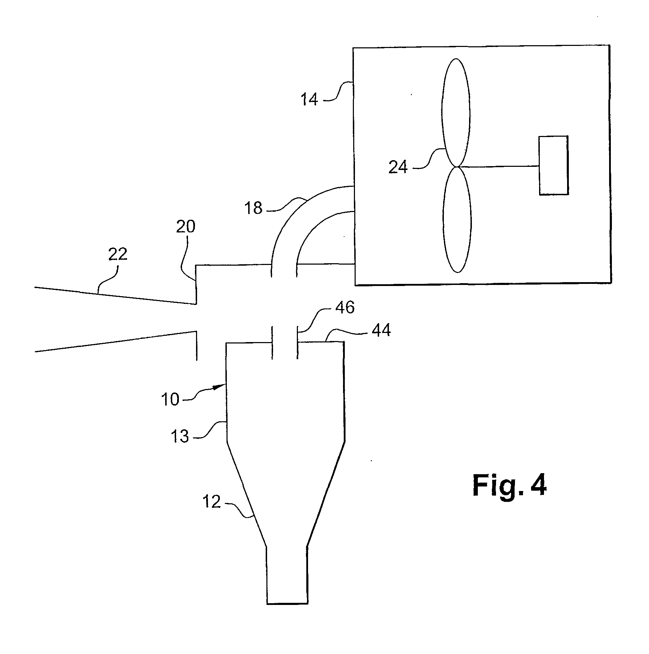

Un tel dispositif est représenté schématiquement en figure 4.

Dans ce cas, le corps cylindrique 13 de l'enceinte 10 est fermé par un couvercle supérieur 44 comportant une sortie d'air axiale 46 éventuellement équipée d'un moyen d'obturation automatique tel qu'une membrane en caoutchouc ou un joint d'étanchéité par exemple. Cette enceinte 10 est montée par clipsage ou par rotation quart de tour sur un support fixe 20, ce support comportant, comme dans le mode de réalisation précédent, des moyens 22 d'entrée d'air tangentielle dans l'enceinte 10, un conduit 18 de sortie d'air communiquant avec le volume dans lequel tourne le rotor 24, et une poignée non représentée de tenue et de manipulation du dispositif.

Après un prélèvement, l'enceinte 10 est détachée du support 20, l'orifice d'entrée d'air dans l'enceinte est obturé, puis une quantité déterminée d'un liquide approprié peut être injectée dans l'enceinte 10 par le conduit axial supérieur 46 pour mettre en solution les particules et micro-organismes déposés sur les parois internes du corps cylindrique 13 et de la partie inférieure 12 de l'enceinte.

De façon générale, le dispositif selon l'invention permet de récupérer un échantillon représentatif des particules et micro-organismes présents dans l'air ambiant par prélèvement d'un volume d'air ambiant relativement important.

Les échantillons prélevés sont conditionnés dans les enceintes cylindriques 10 démontées du dispositif et sont protégés contre les risques de contamination.

Le facteur de concentration des particules et micro-organismes prélevés qui sont en solution dans le liquide injecté est adaptable à volonté. Les durées de prélèvement sont réglables.

Les échantillons collectés sont séparés les uns des autres dans les diverses enceintes cylindriques 10, ce qui évite tout risque de contamination entre ces échantillons.

En outre, l'obtention d'échantillons sous forme liquide permet d'appliquer de nouvelles méthodes d'analyse basées sur des techniques de biologie moléculaire, de microscopies en épi-fluorescence, de cytométrie en flux, de bioluminescence, d'immunologie ou de chromotographie. On peut ainsi évaluer la flore microbienne totale de l'air ambiant et/ou la flore microbienne viable, sans être limité à l'évaluation de la flore cultivable qui ne représente que 0,1 à 10 % environ de la flore microbienne totale.

Les nouvelles techniques d'analyse auxquelles on peut soumettre les échantillons prélevés au moyen du dispositif selon l'invention permettent également de rechercher spécifiquement des bactéries ou des moisissures ou bien de sonder une espèce en particulier.

De plus, le dispositif selon l'invention et la mallette précitée contenant des accessoires tels que des enceintes amovibles, un ou plusieurs réservoirs de liquide d'injection, un ou des accumulateurs rechargeables, éventuellement des moyens de recharge d'accumulateurs, etc., constituent un kit autonome et portable de collecte et de séparation de particules et de micro-organismes présents dans l'air ambiant, qui trouve des applications dans des domaines très nombreux et très divers.

## Revendications

1. Dispositif de collecte et de séparation de particules et de micro-organismes présents dans l'air ambiant, comprenant des moyens (14) d'aspiration d'air dans une enceinte (10) de centrifugation qui comporte des moyens de retenue de particules ou de micro-organismes et des moyens (18) de sortie de l'air aspiré, **caractérisé en ce que** l'enceinte (10) est montée de façon amovible sur le dispositif et forme un récipient de transport des particules et micro-organismes collectés, et comprend essentiellement une chambre cylindrique de volume compris entre 100 et 200 cm³ et dans laquelle les particules et micro-organismes véhiculés par l'air aspiré sont mis en rotation autour de l'axe de l'enceinte et sont séparés de l'air aspiré par centrifugation sur la paroi interne de l'enceinte, cette enceinte constituant avec les moyens (14) d'aspiration d'air un ensemble portable autonome et manipulable d'une seule main, le débit d'air aspiré dans ladite enceinte par les moyens d'aspiration étant compris entre 200 et 400 litres par minute environ.

2. Dispositif selon la revendication 1, **caractérisé en ce que** l'enceinte (10) comprend des moyens d'obturation automatique de son extrémité supérieure, qui sont ouverts quand l'enceinte est montée sur le dispositif et qui sont fermés quand l'enceinte est retirée du dispositif.

3. Dispositif selon la revendication 2, **caractérisé en ce que** les moyens de fermeture automatique comprennent un diaphragme annulaire et des moyens de rappel élastique du diaphragme en partie de fermeture.

4. Dispositif selon l'une des revendications précédentes, **caractérisé en ce que** l'enceinte (10) est montée sur le dispositif par encliquetage élastique ou par rotation quart de tour.

5. Dispositif selon l'une des revendications 1 à 4, **caractérisé en ce que** l'enceinte (10) est montée sur une paroi supérieure (11) fixe comportant une sortie d'air axiale (18) et une entrée d'air tangentielle (22).

6. Dispositif selon l'une des revendications 1 à 5, **caractérisé en ce qu'**il comprend des moyens (34) d'injection de liquide en partie supérieure (11,20) de ladite enceinte.

7. Dispositif selon la revendication 6, **caractérisé en ce que** les moyens (34) d'injection de liquide débouchent dans un conduit (22) d'entrée d'air dans ladite enceinte.

8. Dispositif selon l'ensemble des revendications 5 et 6, **caractérisé en ce que** les moyens (34) d'injection de liquide comprennent plusieurs orifices débouchant dans ladite enceinte, répartis de façon régulière sur une circonférence d'un rebord (20) de la paroi supérieure fixe (11).

9. Dispositif selon l'une des revendications 5 à 8, **caractérisé en ce que** ladite sortie axiale (18) de l'enceinte est destinée à être reliée à une prise d'aspiration d'air.

10. Dispositif selon l'une des revendications 5 à 8, **caractérisé en ce que** les moyens d'aspiration d'air (14) sont solidaires de la paroi supérieure fixe (11,20) et aspirent l'air par la sortie axiale (18) de l'enceinte.

11. Dispositif selon l'une des revendications précédentes, **caractérisé en ce qu'**il comprend une poignée (16) contenant un réservoir de liquide (30), des moyens (36) de mise en pression du liquide dans le réservoir, et des moyens de liaison du réservoir à des moyens (34) d'injection dans l'enceinte précitée.

12. Dispositif selon la revendication 11, **caractérisé en ce que** lesdits moyens de liaison comprennent des moyens (32) de réglage du débit de liquide vers des moyens d'injection (34).

13. Dispositif selon la revendication 11 ou 12, **caractérisé en ce que** la poignée (16) comprend des moyens (40) de commande des moyens (14) d'aspiration d'air.

14. Dispositif selon l'une des revendications précédentes, **caractérisé en ce que** l'enceinte amovible (10) est à usage unique ou est réalisée en matériau autoclavable.

15. Dispositif selon l'une des revendications précédentes, **caractérisé en ce qu'**il est destiné à la collecte et la séparation de particules et micro-organismes ayant des dimensions comprises entre 0,5 et 15 µm environ.

16. Dispositif selon l'une des revendications précédentes, **caractérisé en ce que** les moyens (18) de sortie d'air de ladite enceinte comprennent un filtre de capture des particules et micro-organismes ayant des dimensions inférieures à 1 µm environ.

17. Kit autonome et portable de séparation et de collecte de particules et de micro-organismes présents dans l'air ambiant, **caractérisé en ce qu'**il comprend un dispositif du type décrit dans l'une des revendications précédentes et une mallette d'accessoires tels par exemple que des enceintes amovibles de rechange, au moins un réservoir de liquide d'injection et au moins un accumulateur électrique rechargeable.

## Patentansprüche

1. Vorrichtung zum Sammeln und Trennen von in der Umgebungsluft vorhandenen Partikeln und Mikroorganismen, enthaltend Mittel (14) zum Ansaugen von Luft in ein Zentrifugiergefäß (10), das Mittel zum Zurückhalten von Partikeln bzw. Mikroorganismen aufweist, und Mittel (18) zum Ausströmen der angesaugten Luft, **dadurch gekennzeichnet, dass** das Gefäß (10) an der Vorrichtung abnehmbar montiert ist und einen Behälter zum Transportieren der gesammelten Partikel und Mikroorganismen bildet und im Wesentlichen eine zylindrische Kammer mit einem Volumen von 100 bis 200 cm³ enthält, in welche die mit der angesaugten Luft mitgerissenen Partikel und Mikroorganismen in Drehung um die Achse des Gefäßes versetzt werden und durch Zentrifugieren an der Innenwand des Gefäßes von der angesaugten Luft getrennt werden, wobei dieses Gefäß mit den Mitteln (14) zum Ansaugen von Luft eine eigenständige, tragbare und mit nur einer Hand handhabbare Einheit bildet, wobei die Luftdurchsatzmenge der von den Ansaugmitteln in das Gefäß angesaugten Luft zwischen etwa 200 und 400 Liter pro Minute beträgt.

2. Vorrichtung nach Anspruch 1, **dadurch gekennzeichnet, dass** das Gefäß (10) Mittel zum automatischen Verschließen seines oberen Endes aufweist, die dann offen sind, wenn das Gefäß an der Vorrichtung montiert ist, und dann geschlossen sind, wenn das Gefäß von der Vorrichtung abgenommen ist.

3. Vorrichtung nach Anspruch 2, **dadurch gekennzeichnet, dass** die Mittel zum automatischen Verschließen eine ringförmige Membrane und Mittel zum elastischen Rückstellen der Membrane in den Schließbereich aufweisen.

4. Vorrichtung nach einem der vorangehenden Ansprüche, **dadurch gekennzeichnet, dass** das Gefäß (10) durch elastisches Einrasten oder durch eine Viertelumdrehung an der Vorrichtung montiert ist.

5. Vorrichtung nach einem der Ansprüche 1 bis 4, **dadurch gekennzeichnet, dass** das Gefäß (10) an einer festen oberen Wand (11) montiert ist, die einen axialen Luftauslass (18) und einen tangentialen Lufteinlass (22) aufweist.

6. Vorrichtung nach einem der Ansprüche 1 bis 5, **dadurch gekennzeichnet, dass** sie Mittel (34) zum Einspritzen von Flüssigkeit in den oberen Bereich (11, 20) des Gefäßes aufweist.

7. Vorrichtung nach Anspruch 6, **dadurch gekennzeichnet, dass** die Mittel (34) zum Einspritzen von Flüssigkeit in einen Kanal (22) zum Einströmen von Luft in das Gefäß münden.

8. Vorrichtung nach Anspruch 5 und 6 zusammengenommen, **dadurch gekennzeichnet, dass** die Mittel (34) zum Einspritzen von Flüssigkeit mehrere Öffnungen aufweisen, die in das Gefäß münden und gleichmäßig über einen Umfang einer Randkante (20) der festen oberen Wand (11) verteilt sind.

9. Vorrichtung nach einem der Ansprüche 5 bis 8, **dadurch gekennzeichnet, dass** der axiale Auslass (18) des Gefäßes dazu bestimmt ist, mit einem Anschluss zum Absaugen von Luft verbunden zu werden.

10. Vorrichtung nach einem der Ansprüche 5 bis 8, **dadurch gekennzeichnet, dass** die Mittel (14) zum Ansaugen von Luft fest mit der festen oberen Wand (11, 20) verbunden sind und Luft durch den axialen Auslass (18) des Gefäßes absaugen.

11. Vorrichtung nach einem der vorangehenden Ansprüche, **dadurch gekennzeichnet, dass** sie einen Griff (16) aufweist, der einen Flüssigkeitsvorratsbehälter (30), Mittel (36) zum Unterdrucksetzen der Flüssigkeit in dem Vorratsbehälter und Mittel zum Verbinden des Vorratsbehälters mit Mitteln (34) zum Einspritzen in das vorgenannte Gefäß beinhaltet.

12. Vorrichtung nach Anspruch 11, **dadurch gekennzeichnet, dass** die Verbindungsmittel Mittel (32) zum Einstellen der Durchsatzmenge an Flüssigkeit zu den Einspritzmitteln (34) aufweisen.

13. Vorrichtung nach Anspruch 11 oder 12, **dadurch gekennzeichnet, dass** der Griff (16) Mittel (40) zum Steuern der Luftansaugmittel (14) aufweist.

14. Vorrichtung nach einem der vorangehenden Ansprüche, **dadurch gekennzeichnet, dass** das abnehmbare Gefäß (10) zum einmaligen Gebrauch bestimmt ist oder aus einem autoklavierbaren Material besteht.

15. Vorrichtung nach einem der vorangehenden Ansprüche, **dadurch gekennzeichnet, dass** sie zum Sammeln und Trennen von Partikeln und Mikroorganismen mit Abmessungen zwischen etwa 0,5 und 15 µm bestimmt ist.

16. Vorrichtung nach einem der vorangehenden Ansprüche, **dadurch gekennzeichnet, dass** die Mittel (18) zum Ausströmen von Luft aus dem Gefäß ein Filter zum Abfangen von Partikeln und Mikroorganismen mit Abmessungen von unter etwa 1 µm aufweisen.

17. Eigenständiger und tragbarer Teilesatz zum Trennen und Sammeln von in der Umgebungsluft vorhandenen Partikeln und Mikroorganismen, **dadurch gekennzeichnet, dass** er eine Vorrichtung vom Typ wie in einem der vorangehenden Ansprüche beschrieben und einen Koffer mit Zubehör, wie beispielsweise abnehmbare Austauschgefäße, zumindest einen Vorratsbehälter für Einspritzflüssigkeit und zumindest einen wiederaufladbaren elektrischen Akkumulator enthält.

## Claims

1. A device for collecting and separating particles and microorganisms present in ambient air, the device comprising means (14) for sucking air into a centrifugal enclosure (10) having means for retaining particles or microorganisms and means (18) for exhausting of the sucked-in air, the device being **characterized in that** the enclosure (10) is removably mounted on the device and forms a receptacle for transporting collected particles and microorganisms, and essentially comprises a cylindrical chamber having a volume lying in the range about 100 to 200 cm³, in which the particules and microorganisms conveyed by the sucked-in air are set into rotation about the axis of the enclosure and are separated from the sucked-in air by centrifuging against the inside wall of the enclosure, said enclosure cooperating with the air suction means (14) to constitute a self-contained, portable assembly that can be manipulated using only one hand, the flow rate of air sucked into said enclosure by the suction means (14)lying in the range about 200 L/min to 400 L/min.

2. A device according to claim 1, **characterized in that** the enclosure (10) includes automatic closure means for closing its top end, which means are open while the enclosure is mounted on the device and are closed while the enclosure is separate from the device.

3. A device according to claim 2, **characterized in that** the automatic closure means comprise an annular diaphragm, and resilient means for urging the diaphragm towards its shut position.

4. A device according to any preceding claim, **characterized in that** the enclosure (10) is mounted on the device by resilient snap-fastening or by turning through one-fourth of a turn.

5. A device according to any one of claims 1 to 4, **characterized in that** the enclosure (10) is mounted on a main top wall (11) including an axial air outlet (18) and a tangential air inlet (22).

6. A device according to any one of claims 1 to 5, **characterized in that** it includes means (34) for injecting liquid into the top portion (11, 20) of said enclosure.

7. A device according to claim 6, **characterized in that** the means (34) for injecting liquid open out into a duct (32) for admitting air into said enclosure.

8. A device according to claims 5 and 6 taken together, the device being **characterized in that** the liquid injection means (34) comprise a plurality of orifices opening out into said enclosure, that are regularly distributed around a circumference of a rim (20) of the main top wall (11).

9. A device according to claims 5 to 8, **characterized in that** said axial outlet (18) of the enclosure is designed to be connected to an air suction socket.

10. A device according to any one of claims 5 to 8, **characterized in that** the air suction means (14) are secured to the main top wall (11, 20) and suck in air through the axial outlet (18) of the enclosure.

11. A device according to any preceding claim, **characterized in that** it includes a handle (16) containing a tank (30) of liquid, means (36) for putting the liquid in the tank under pressure, and means for connecting the tank to means (34) for injecting the liquid into the above-mentioned enclosure.

12. A device according to claim 11, **characterized in that** said connection means include means (32) for adjusting the flow rate of liquid towards the injection means (34).

13. A device according to claim 11 of claim 12, **characterized in that** the handle (16) includes control means (40) for controlling the air suction means (14).

14. A device according to any preceding claim, **characterized in that** the removable enclosure (10) is for single use, or is made of a material suitable for putting in an autoclave.

15. A device according to any preceding claim, **characterized in that** it is for collecting and separating particles and microorganisms having dimensions lying in the range about 0.5 µm to about 15 µm.

16. A device according to any preceding claim, **characterized in that** the means (18) for exhausting air from said enclosure include a filter for capturing particles and microorganisms of dimensions smaller than about 1 µm.

17. A self-contained and portable kit for separating and collecting particles and microorganisms present in ambient air, the kit being **characterized in that** it comprises a device of the type described in any preceding claim, together with a bag of accessories comprising: removable spare enclosures; at least one tank of injection liquid; and at least one rechargeable battery.
